# FASCICULE DE BREVET EUROPEEN

(11) **EP 1 216 061 B1**
(45) Date de publication et mention de la délivrance du brevet: **24.03.2004**
(21) Numéro de dépôt: 00993307.8
(22) Date de dépôt: 07.12.2000
(51) Int. Cl.: A61L 2/00

(54) **PROCEDE ET DISPOSITIF VIBRATOIRE DE CONDITIONNEMENT, DE CLIMATISATION, DE REFROIDISSEMENT ET DE DECONTAMINATION, DE DESINFECTION, DE STERILISATION DE MILIEUX PHYSIQUES**
VERFAHREN UND VIBRATIONSVORRICHTUNG ZUR KONDITIONIERUNG, KLIMATISIERUNG, KÜHLUNG UND DEKONTAMINIERUNG, DESINFEKTION UND STERILISIERUNG PHYSIKALISCHER MEDIEN
METHOD AND VIBRATING DEVICE FOR CONDITIONING, AIR-CONDITIONING, COOLING AND DECONTAMINATING, DISINFECTING AND STERILIZING PHYSICAL MEDIA

(30) Priorité: 10.12.1999 FR 9915584
(43) Date de publication de la demande: 26.06.2002
(73) Titulaire: Touzova, Tamara, 75012 Paris (FR); DOUBOCHINSKI, Danil, 75012 Paris (FR)
(72) Inventeur: DOUBOCHINSKI, Daniel, F-75012 Paris (FR); PRYTKOV, Roman, 220090 MINSK (BY)
(74) Mandataire: Jacobson, Claude
(86) Numéro de dépôt international: PCT/FR2000/003439
(87) Numéro de publication internationale: WO 2001/041817

(56) Documents cités:
- US-A- 5 527 459
- PATENT ABSTRACTS OF JAPAN vol. 012, no. 372 (C-533), 5 octobre 1988 (1988-10-05) & JP 63 123343 A (NISSEI REIKI KK), 27 mai 1988 (1988-05-27)
- PATENT ABSTRACTS OF JAPAN vol. 003, no. 137 (M-080), 14 novembre 1979 (1979-11-14) -& JP 54 111161 A (MATSUSHITA ELECTRIC IND CO LTD), 31 août 1979 (1979-08-31)
- PATENT ABSTRACTS OF JAPAN vol. 012, no. 176 (C-498), 25 mai 1988 (1988-05-25) & JP 62 282686 A (IWASAKI ELECTRIC CO LTD), 8 décembre 1987 (1987-12-08)

## Description

L'invention est relative au domaine du conditionnement, de la climatisation, du refroidissement et de la décontamination, de la désinfection, de la stérilisation de milieux physiques absorbants du froid au moyen d'actions périodiques ou vibratoires et concerne un procédé et des dispositifs permettant d'assurer de telles fonctions.

L'invention est applicable aux milieux liquides, gazeux et à des milieux mélangés dans la composition desquels peuvent entrer des liquides, des gaz et des substances colloïdales et solides. Les éléments traités par les actions périodiques et les éléments réalisant des échanges calorifiques peuvent être représentés par des agents frigorifiques constitués par des milieux liquides, gazeux, solides et mixtes, organiques et/ou non organiques, tandis que les actions périodiques réalisant du conditionnement, de la climatisation, du refroidissement et de la décontamination, de la désinfection, de la stérilisation de milieux physiques précités peuvent être représentés par des systèmes vibratoires mécaniques, électromécaniques, ultrasonores, magnétiques, électromagnétique et mixtes.

On connaît (voir p.ex. US-A-5 527 459) des procédés et des dispositifs de conditionnement, de climatisation, de refroidissement et de décontamination, de désinfection, de stérilisation de milieux physiques absorbants du froid utilisés dans les climatiseurs, conditionneurs, appareils frigorifiques et systèmes de décontamination actuels.

Les inconvénients essentiels des procédés et dispositifs existants sont les suivants : dépenses d'énergie importantes ; efficacité et rendement faibles ; construction complexe ; durée de cycle importante ; coût élevé des installations et de leur exploitation ; pollution et bruit important ; importants volume et surface nécessaires pour la réalisation de ces fonctions ; faible niveau de protection contre la contamination - ce qui en fin de compte limite leurs applications pratiques.

Le procédé proposé et le dispositif pour sa mise en oeuvre, sont dépourvus des inconvénients indiqués plus haut, simplifient de façon importante les principes et les systèmes qui les réalisent, accroissent de façon importante le rendement des dispositifs et diminuent de plusieurs fois les dépenses en temps et en énergie pour la réalisation des traitements de conditionnement, de climatisation, de refroidissement et de décontamination, de désinfection, de stérilisation de milieux physiques.

Elle a donc pour objet un procédé vibratoire de conditionnement, de climatisation, de refroidissement et de décontamination, de désinfection, de stérilisation de milieux physiques, tel que defini dans la revendication 1.

D'autres caractéristiques de l'invention sont décrites dans les revendications 2 et 3.

L'invention a en outre pour objet un dispositif pour la mise en oeuvre du procédé défini ci-dessus, tel que défini dans la revendication 4.

Suivant d'autres caractéristiques :
- les premier et second systèmes sont réunis en un seul système d'action périodique,
- les premier et second systèmes d'action périodique sont mis en mouvement par l'énergie du courant régulé du milieu physique notamment lorsque les premier et second systèmes sont réalisés sous forme de sirène ou de sifflet,
- la chambre frigorifique et le changeur de chaleur sont réunis en un seul système d'échange de chaleur,
- les systèmes de régulation du débit des milieux physiques sont constitués par des vannes régulées et/ou des pompes aspirantes.

L'invention a enfin pour objet, un dispositif pour la mise en oeuvre du procédé défini ci-dessus, caractérisé en ce qu'il comporte au moins un premier système d'action vibratoire notamment de rayonnement électromagnétique et/ou thermique, qui agit sur le courant régulé du milieu physique auxiliaire, au moins un second système d'action périodique, notamment de type mécanique, pneumatique, hydraulique, magnétique, électrique, ultrasonore ou mixte assurant la micronisation du milieu physique auxiliaire dans une chambre de traitement, par exemple une chambre frigorifique et/ou une chambre acoustique, ou encore chambre d'échange de chaleur (échangeur de chaleur) ou mixte, et le pulvérisant sous forme de vapeur ou d'aérosol à l'aide d'un système de pulvérisation, formant au moins un système de refroidissement et/ou de congélation du milieu réfrigérant, au moins un premier circuit fermé de circulation du milieu physique réfrigérant, au moins un système de régulation de son débit à l'aide d'un premier dispositif de régulation constitué d'une vanne régulée et/ou d'une pompe aspirante, au moins un second circuit fermé de circulation du milieu physique absorbant du froid, au moins un système de régulation de son débit à l'aide d'un second dispositif de régulation constitué d'une vanne régulée et/ou d'une pompe aspirante, au moins une chambre frigorifique et au moins un échangeur de chaleur réalisant l'interaction entre le milieu réfrigérant et le milieu absorbant du froid, et au moins un système vibratoire de décontamination, de désinfection et de stérilisation du milieu physique absorbant du froid.

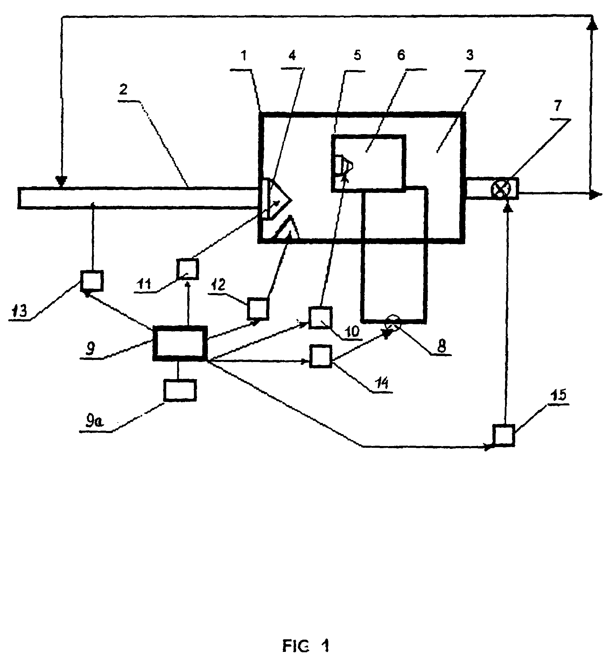

Sur la figure 1 est représenté schématiquement le schéma de principe d'un dispositif mettant en oeuvre le procédé proposé de conditionnement, de climatisation, de refroidissement et de décontamination, de désinfection, de stérilisation de milieux physiques.

Le dispositif représenté à la figure 1 comporte un système d'échange de chaleur (échangeur de chaleur) 1 dans lequel un courant régulé d'entrée d'un milieu physique auxiliaire 2 est refroidi et forme un milieu réfrigérant 3, un système de traitement vibratoire 4, au moins une chambre 5 constituant un volume dans lequel s'accumule le milieu 6 absorbant du froid, un dispositif 7 assurant l'évacuation et/ou la circulation régulée du courant du milieu réfrigérant 3, se présentant par exemple sous la forme d'une vanne régulée et/ou d'une pompe d'évacuation, un dispositif 8 assurant un courant régulé du milieu 6 absorbant du froid, constitué par exemple par une vanne régulée et/ou une pompe d'évacuation, au moins une source 9 d'alimentation réglable par moyen de réglage ou régulateur électronique 9a d'action périodique externe (vibratoire), des systèmes 13, 12, 10, et 11 assurant des actions vibratoires respectivement sur le courant d'entrée du milieu physique 2, sur le milieu réfrigérant 3, sur le milieu 6 absorbant du froid et sur le système 4 de traitement vibratoire du milieu physique 2 et au moins un système 15, 14 assurant l'évacuation et/ou la circulation régulée du courant du milieu réfrigérant 3 et du courant du milieu absorbant du froid 6 par les dispositifs 7 et 8.

Le milieu physique 2 peut être constitué d'un milieu liquide, gazeux ou mélangé avec des substances organiques et/ou non organiques, dans la composition duquel peuvent entrer des liquides, des gaz, des corps colloïdaux ou solides ou leurs combinaisons.

Le milieu absorbant du froid 6 peut être constitué d'un milieu liquide, gazeux ou mélangé avec des substances organiques et/ou non organiques de type liquide, gazeux, colloïdal et solide ou de combinaisons de telles substances.

Les systèmes d'action vibratoire 11,12,13 peuvent se présenter sous la forme d'au moins un dispositif d'action ultrasonore, par exemple une sirène, un sifflet, un vibrateur piézo-électrique ou leur combinaison, d'au moins un dispositif de type à magnétostriction, un dispositif de type magnétique ou électromagnétique, créant par exemple un champ magnétique ou électromagnétique de configuration ou d'intensité correspondante, ou, enfin, de différentes combinaisons des dispositifs et systèmes énumérés plus haut. Le systèmes d'action vibratoire 10 peut se présenter sous la forme d'au moins un dispositif magnétique ou électromagnétique, créant par exemple un champ magnétique ou électromagnétique de configuration ou d'intensité correspondante au besoin de décontamination, de désinfection, de stérilisation de milieux physiques. Le système 15 assure l'évacuation et/ou la circulation régulée du courant du milieu réfrigérant 3 par le dispositif 7. Le système 14 assure l'évacuation et/ou la circulation régulée du courant régulée du milieu absorbant du froid 6 par le dispositif 8. La source d'alimentation 9 assure les régimes de fonctionnement indispensables des systèmes 10,11,12,13,14 et 15 et comprend le régulateur 9a permettant de faire varier les fréquences et les amplitudes d'énergie en fonction des régimes de fonctionnement de ces systèmes. De cette manière les systèmes 9 et 9a peuvent assurer la régulation du débit du dispositif par variation de la fréquence et de l'amplitude des actions périodiques sur les milieux traités 2, 3 et sur les dispositifs de régulation de l'évacuation et de la circulation de ces milieux. En qualité de source d'énergie pour alimenter le système 4, on peut utiliser le courant du milieu traité 2.

Les actions vibratoires 11, 12, 13 peuvent être assurées par un seul et même vibrateur ou système.

Le dispositif de traitement vibratoire 4 peut comporter :
- au moins un dispositif de micronisation et de pulvérisation à fine dispersion du milieu physique 2, par exemple un pulvérisateur mécanique 4 et/ou acoustique, et/ou pneumatique, et/ou encore hydraulique ou mixte, qui disperse et injecte le milieu physique 2 sous forme de vapeur ou d'aérosol avec des dimensions de particules (gouttelettes) du niveau moléculaire (inférieures au micron).

Dans ce cas, le processus traditionnel coûteux et consommant de l'énergie est remplacé par une technologie d'injection à faible dispersion qui se caractérise par une demande d'énergie et de temps des dizaines et des centaines de fois plus faible.

Ainsi, la chambre 1 peut être constituée d'une chambre acoustique de microniseur, le dispositif de traitement vibratoire 4 est un système comprenant un injecteur qui pulvérise le milieu physique 2 par dispersion fine.

Conformément au schéma représenté à la figure 1, le procédé vibratoire de conditionnement, de climatisation, de refroidissement et de décontamination, de désinfection, de stérilisation de milieu physique est mis en oeuvre de la façon suivante.

Le courant régulé du milieu physique 2 pénètre dans la chambre 1 du système d'échange du chaleur.

Au cours de son déplacement, le milieu physique est soumis à au moins une action périodique 11 immédiatement avant son introduction dans la chambre 1 ainsi que dans la chambre 1 elle-même à l'aide du système 12. Les actions 11 et 12 précitées créent les conditions de refroidissement du milieu 2 sous l'action des vibrations mécaniques, pneumatique, hydraulique, magnétique, électromagnétiques, acoustiques ou/et mixte, et contribuent à son évacuation efficace à l'aide du système 7 en régime régulé.

Dans la chambre 5, le milieu physique absorbant du froid 6 entre en interaction avec le milieu réfrigérant 3 auquel est appliquée une action vibratoire, assurant les conditions de son refroidissement et de son fonctionnement ininterrompu en régime stationnaire.

Le courant régulé du milieu physique absorbant de la chaleur 6 circule par exemple en circuit stationnaire. Au cours de son déplacement, le courant de milieu physique 6 peut être soumis à au moins une action vibratoire 10 par exemple au moyen d'un rayonnement à micro-ondes, ce qui crée une condition complémentaire favorable à la décontamination, désinfection et stérilisation de milieu physique 6.

Les actions 11 et 13 précitées établissent les conditions nécessaires et suffisantes pour une pulvérisation du milieu physique 2 à dispersion fine, sous forme de vapeur et/ou d'aérosol, et elles le préparent pour le processus de refroidissement rapide et/ou de congélation dans le système 1.

Les particules à dispersion fine du milieu réfrigérant 3 refroidies et/ou congelées, par exemple d'eau pure, pénètrent dans la chambre 1 dans laquelle elles fondent sous l'action d'un rayonnement périodique 12, par exemple à micro-ondes ou d'un autre rayonnement thermique ou électromagnétique, et sont évacuées ou/et circulent à l'aide du système 7 en régime régulé.

La présente invention fait appel à un procédé d'action simultanée sur un milieu physique 2 (Fig.1), et/ou sur un milieu absorbant de la chaleur 6, à l'aide d'au moins deux actions périodiques, comme décrit précédemment dans le brevet français FR-A-2 744 931 des mêmes demandeurs.

Cependant, à la différence de l'invention décrite dans la demande de brevet précitée, la mise en oeuvre d'au moins deux actions périodiques sur les milieux traités est utilisée non pas pour leur mélange, mais pour leur vaporisation froide et leur refroidissement.

Dans le cadre de la présente invention, le procédé selon le brevet français FR-A-2 744 931 est appliqué à des traitements de conditionnement, climatisation, refroidissement et décontamination, désinfection, stérilisation de milieux physiques de la façon suivante.

Conformément au procédé décrit dans le brevet français n° 96 02 080, dans des conditions déterminées, dans un milieu traité simultanément par au moins deux actions périodiques, on réalise un traitement vibratoire, qui brise les particules et molécules du milieu physique 2 (Fig.1), en constituants, par exemple en molécules de gaz, pour former l'aérosol et/ou la vapeur du milieu réfrigérant 3 (Fig.1).

Les molécules du milieu réfrigérant 3 micronisées forment une grande surface, consomment de l'énergie calorifique apportée par le milieu 6, et changent ses états d'agrégation. Cette interaction permet de réaliser l'échange de chaleur entre les milieux 3 et 6.

Ainsi, le processus de traitement vibratoire et de circulation des différents milieux par action sur eux d'une série d'actions périodiques permet de réaliser leur utilisation à l'aide des dispositifs d'évacuation 7 et 8 (Fig.1).

## Revendications

1. Procédé vibratoire de conditionnement, de climatisation, de refroidissement et de décontamination, de désinfection, de stérilisation d'un milieu physique à traiter absorbant du froid (6), **caractérisé en ce que** :
- on agit, à l'aide d'au moins une première action périodique (13), sur un courant d'entrée régulé d'un milieu physique auxiliaire (2) comprenant des particules et molécules, constituant un agent frigorifique, et, à l'aide d'au moins une seconde action périodique (11), sur le même milieu physique dans une chambre frigorifique (1), la fréquence de la deuxième action périodique différant de la fréquence de la première action périodique d'au moins un facteur 10, on module à l'aide de cette seconde action périodique (11), ladite première action périodique par une modulation de fréquence, de phase et/ou d'amplitude, ou par une modulation combinée, on agit par résonance à l'aide des composantes spectrales qui se sont formées du fait de la modulation, sur les particules et molécules du milieu physique auxiliaire (2) dont les fréquences propres sont égales et/ou correspondent dans l'ensemble aux fréquences des composantes spectrales précitées et on réalise ainsi un phénomène de cavitation résonnante qui brise les particules et molécules du milieu physique 2, en constituants, par exemple en molécules de gaz, pour former l'aérosol et/ou la vapeur du milieu réfrigérant 3 et, de ce fait, on forme un flux de milieu physique réfrigérant (3) et on crée les conditions nécessaires et suffisantes de son refroidissement,
- on forme une interaction entre le milieu physique réfrigérant (3) et le milieu physique à traiter, absorbant du froid (6), dans un système d'échange de chaleur (1 ),
- on agit à l'aide d'au moins une troisième action périodique (10) sur le milieu absorbant du froid (6) et de ce fait, on décontamine de façon continue ou/et périodique ce milieu physique et on crée les conditions nécessaires et suffisantes du fonctionnement du système de décontamination en régime stationnaire de fonctionnement,
- on agit à l'aide d'au moins une quatrième action (15) sur le milieu réfrigérant (3) et de ce fait, on réalise ainsi les conditions nécessaires et suffisantes pour former au moins un premier circuit fermé de circulation du milieu physique réfrigérant (3) par régulation de son débit à l'aide d'un premier dispositif de régulation (7),
- on agit à l'aide d'au moins une cinquième action (14) sur le milieu absorbant du froid (6) et de ce fait, on réalise ainsi les conditions nécessaires et suffisantes pour former au moins un second circuit fermé de circulation du milieu physique absorbant du froid (6) par régulation de son débit à l'aide d'un second dispositif de régulation (8),
- on alimente les systèmes d'action (11 à 15) par de l'énergie d'au moins une source (9) et d'au moins un régulateur (9a) en régime optimal et on réalise ainsi les conditions nécessaires et suffisantes du traitement vibratoire desdits milieux (2, 3 et 6) et on assure des régimes efficaces de fonctionnement régulé du système de conditionnement, de climatisation, de refroidissement et de décontamination, de désinfection, de stérilisation du milieu physique à traiter (6).

2. Procédé suivant la revendication 1, **caractérisé en ce que** :
- à l'aide de la seconde action périodique (11), de nature mécanique, pneumatique, hydraulique, électrique, magnétique, électromagnétique, acoustique, ou mixte, on micronise et on divise le milieu physique auxiliaire (2) et, par modification de son état d'agrégation, on obtient le milieu réfrigérant (3) sous forme de vapeur et/ou d'aérosol avec des dimensions de particules inférieures au micron, et
- à l'aide de la quatrième action (15), qui est une action périodique, sur le milieu physique réfrigérant micronisé (3), on récupère ce milieu pour l'utiliser dans ledit premier circuit fermé à l'aide dudit premier dispositif de régulation (7).

3. Procédé suivant l'une des revendications 1 et 2, **caractérisé en ce que** :
- on agit de façon complémentaire sur le courant du milieu réfrigérant (3) par la première action périodique (11), notamment par un rayonnement dur ou par une radiation magnétique, ou encore par un rayonnement électromagnétique et/ou mixte, afin de créer ainsi des conditions complémentaires favorisant sa décontamination, sa désinfection et sa stérilisation.

4. Dispositif pour la mise en oeuvre du procédé suivant la revendication 1, **caractérisé en ce qu'**il comporte au moins un premier système d'action périodique (13) et au moins un second système d'action périodique (11) alimentés par une source d'énergie (9) et agissant sur un courant régulé du milieu physique (2) constituant le milieu physique réfrigérant (3) dans une chambre frigorifique (1), au moins un premier circuit fermé de circulation du milieu physique réfrigérant (3), au moins un système de régulation de son débit à l'aide d'un dispositif de régulation (7), au moins un second circuit fermé de circulation du milieu physique absorbant du froid (6), au moins un système de régulation de son débit à l'aide d'un dispositif de régulation (8), au moins un échangeur de chaleur (1) réalisant l'interaction entre le milieu réfrigérant (3) et le milieu absorbant du froid (6), et au moins un système vibratoire (10) de décontamination, de désinfection, de stérilisation du milieu physique absorbant du froid.

5. Dispositif suivant la revendication 4, **caractérisé en ce que** lesdites première et troisième actions vibratoires (13, 11) sont régulées en fréquence et/ou en amplitude en fonction des débits du milieu réfrigérant (3) et du milieu absorbant du froid (6).

## Patentansprüche

1. Schwingungsverfahren der Konditionierung, Klimatisierung, Kühlung und Dekontamination, Desinfektion, Sterilisation eins zu behandelnden kälteabsorbierenden physikalischen Mediums (6), **dadurch gekennzeichnet, dass**
man mittels wenigstens eines ersten periodischen Vorgangs (13) auf einen geregelten Eingangsstrom eines physikalischen Hilfsmediums, welches Partikel und Moleküle enthält und ein Kältemittel bildet, und mittels wenigstens eines zweiten periodischen Vorgangs (11) auf das gleiche physikalische Medium in einer Kältekammer (1) einwirkt, wobei die Frequenz des zweiten periodischen Vorgangs sich von der Frequenz des ersten periodischen Vorgangs um mindestens einen Faktor 10 unterscheidet, man mittels dieses zweiten periodischen Vorgangs (11) den ersten periodischen Vorgang durch eine Modulation der Frequenz, der Phase und/oder der Amplitude oder durch eine kombinierte Modulation moduliert, man durch Resonanz mittels Spektralkomponenten, die sich auf Grund der Tatsache der Modulation gebildet haben, auf die Partikel und Moleküle des physikalischen Hilfsmediums (2) einwirkt, deren Eigenfrequenzen den Frequenzen der vorgenannten Spektralkomponenten gleich sind und/oder insgesamt entsprechen, und man so eine resonante Kaviationserscheinung erzeugt, welche die Partikel und Moleküle des physikalischen Mediums (2) in Bestandteile, beispielsweise Gasmoleküle, aufbricht, um das Aerosol und/oder den Dampf des Kühlmediums (3) zu bilden, und man auf Grund dieser Tatsache einen Strom des physikalischen Kühlmediums (3) bildet und die notwendigen und hinreichend Bedingungen für seine Kühlung schafft,
man eine Wechselwirkung zwischen dem physikalischen Kühlmedium (3) und dem zu behandelnden, kälteabsorbierenden physikalischen Medium (6) in einem Wärmeaustauschsystem (1) ausbildet,
man mittels wenigstens eines dritten periodischen Vorgangs (10) auf das kälteabsorbierende Medium (6) einwirkt, und man auf Grund dieser Tatsache in kontinuierlicher und/oder periodischer Weise dieses physikalische Medium dekontaminiert und die notwendigen und hinreichenden Bedingungen für das Arbeiten des Dekontaminationssystems unter stationären Betriebsbedingungen schafft,
man mittels wenigstens eines vierten Vorgangs (15) auf das Kühlmedium (3) einwirkt und man auf Grund dieser Tatsache so die notwendigen und hinreichenden Bedingungen zur Ausbildung wenigstens eines ersten geschlossenen Kreislaufs für das physikalische Kühlmedium (3) durch Regulierung seines Durchsatzes mittels einer ersten Reguliervorrichtung (7) schafft,
man mittels wenigstens eines fünften Vorgangs (14) auf ein kälteabsorbierendes Medium (6) einwirkt, und man auf Grund dieser Tatsache die notwendigen und hinreichenden Bedingungen zur Ausbildung wenigstens eines zweiten geschlossenen Kreislaufs für das kälteabsorbierendes physikalische Medium (6) durch Regulierung seines Durchsatzes mittels einer zweiten Reguliervorrichtung (8) schafft,
man die Vorgangssysteme (11 bis 15) mit Energie aus wenigstens einer Quelle (9) und wenigstens einem Regulierer (9a) im optimalen Bereich speist und man so die notwendigen und hinreichenden Bedingungen für die Schwingungsbehandlung der Medien (2, 3 und 6) verwirklicht und man wirksame regulierte Funktionsbereiche des Systems der Konditionierung, Klimatisierung, Kühlung und Dekontamination, Desinfektion, Sterilisation des zu behandelnden physikalischen Mediums (6) gewährleistet.

2. Verfahren nach Anspruch 1, **dadurch gekennzeichnet, dass**
man mittels des zweiten Schwingungsvorgangs (11), mechanischer, pneumatischer, hydraulischer, elektrischer, magnetischer, elektromagnetischer, akustischer oder gemischter Art, das physikalische Hilfsmedium (2) mikronisiert und teilt und durch Veränderung seines Aggregatzustands das Kühlmedium (3) in Form von Dampf und/oder Aerosol mit Submikronabmessungen der Partikel erhält, und
man mittels des vierten Vorgangs (15), der ein Schwingungsvorgang ist, auf dem mikronisierten physikalischen Kühlmedium (3.) dieses Medium zur Verwendung in dem ersten geschlossenen Kreislauf mittels der ersten Reguliervorrichtung (7) rückgewinnt.

3. Verfahren nach einem der Ansprüche 1 und 2, **dadurch gekennzeichnet, dass**
man in komplementärer Weise auf den Strom des Kühlmittels (3) mit dem ersten Schwingungsvorgang (11), insbesondere mit harter Strahlung oder einer magnetischen Strahlung oder auch einer elektromagnetischen und/oder Mischstrahlung einwirkt, um so komplementäre Bedingungen zu schaffen, die seine Dekontamination, seine Desinfektion oder seine Sterilisation begünstigen.

4. Vorrichtung zur Durchführung des Verfahrens nach Anspruch 1, **dadurch gekennzeichnet, dass** sie wenigstens ein erstes periodisches Vorgangssystem (13) und wenigstens ein zweites periodisches Vorgangssystem (11) , die von einer Energiequelle (9) gespeist werden und auf den geregelten Strom des physikalischen Mediums (2), das das physikalische Kühlmedium (3) in einer Kühlkammer (1) bildet, einwirken, wenigstens einen geschlossenen Kreislauf des physikalischen Kühlmediums (3), wenigstens ein System zur Regulierung seines Durchsatzes mittels einer Reguliervorrichtung (7), wenigstens einen zweiten geschlossenen Kreislauf des kälteabsorbierenden physikalischen Mediums (6), wenigstens ein System zur Regulierung seines Durchsatzes mittels einer Reguliervorrichtung (8), wenigstens einen Wärmetauscher (1), der die Wechselwirkung zwischen dem Kühlmedium und dem kälteabsorbierenden Medium (6) verwirklicht, und wenigstens Schwingungssystem (10) der Dekontamination, Desinfektion, Sterilisation des kälteabsorbierenden physikalischen Mediums aufweist.

5. Vorrichtung nach Anspruch 4, **dadurch gekennzeichnet, dass** der erste und dritte Schwingungsvorgang (13, 11) hinsichtlich Frequenz und/oder Amplitude in Abhängigkeit von den Durchsätzen des Kühlmediums (3) und des kälteabsorbierenden Mediums (6) reguliert sind.

## Claims

1. A vibration method for conditioning, air conditioning, cooling and decontaminating, disinfecting or sterilising a physical medium to be treated which generates heat (6), **characterised in that**,
- with the aid of at least one first periodic action (13), a regulated inlet flow of an auxiliary physical medium (2) comprising particles and molecules forming a refrigerant is acted upon and, with the aid of at least one second periodic action (11), the same physical medium is acted upon in a refrigerating chamber (1), the frequency of the second periodic action differing from the frequency of the first periodic action by at least a factor of 10 and, with the aid of this second periodic action (11), the said first periodic action is modulated by means of a frequency, phase and/or amplitude modulation or a combined modulation and, with the aid of spectral components formed as a result of the modulation, the particles and molecules of the auxiliary physical medium (2) are acted upon by resonance, the natural frequencies thereof being equal and/or corresponding overall to the frequencies of the above-mentioned spectral components, and there is thus produced a phenomenon of resonant cavitation which breaks up the particles and molecules of the physical medium 2 into constituent parts, for example gas molecules, to form the aerosol and/or the vapour of the refrigerant medium 3, and consequently a flow of refrigerant physical medium (3) is formed and the necessary and sufficient conditions for its cooling are established,
- an interaction is created between the refrigerant physical medium (3) and the heat-generating physical medium to be treated (6) in a heat exchange system (1),
- with the aid of at least one third periodic action (10), the heat-generating medium (6) is acted upon and consequently this physical medium is continuously and/or periodically decontaminated and the necessary and sufficient conditions for the decontaminating system to function in a stationary process of functioning are established,
- with the aid of at least one fourth action (15), the refrigerant medium (3) is acted upon and consequently the necessary and sufficient conditions are established for forming at least one closed circuit for the circulation of the refrigerant physical medium (3) by regulating its flow rate with the aid of a first regulating device (7),
- with the aid of at least one fifth action (14), the heat-generating medium (6) is acted upon and consequently the necessary and sufficient conditions are established for forming at least one second closed circuit for the circulation of the heat-generating physical medium (6) by regulating its flow rate with the aid of a second regulating device (8),
- the action systems (11 to 15) are supplied with power from at least one source (9) and at least one regulator (9a) with optimum functioning, and in this way the necessary and sufficient conditions are established for the vibration treatment of the said media (2, 3 and 6) and efficient regulated functioning processes are ensured for the system of conditioning, air conditioning, cooling and decontaminating, disinfecting or sterilising the physical medium (6) to be treated.

2. A method according to Claim 1, **characterised in that**,
- with the aid of the second periodic action (11), which is mechanical, pneumatic, hydraulic, electrical, magnetic, electromagnetic, acoustic or mixed in nature, the auxiliary physical medium (2) is micronised and divided and by modifying its state of aggregation the refrigerant medium (3) is obtained in the form of a vapour and/or aerosol with particle sizes of less than a micron and,
- with the aid of the fourth action (15), which is a periodic action, on the micronised refrigerant physical medium (3), this medium is recovered for use in the said first closed circuit with the aid of the said first regulating device (7).

3. A method according to either of Claims 1 and 2, **characterised in that**
- the flow of refrigerant medium (3) is acted upon in complementary manner by the first periodic action (11), in particular by hard radiation or magnetic radiation, or indeed by electromagnetic and/or mixed radiation, in order to establish in this way complementary conditions favouring its decontamination, disinfection and sterilisation.

4. A device for carrying out the method according to Claim 1, **characterised in that** it has at least one first system of periodic action (13) and at least one second system of periodic action (11) which are supplied with a source of power (9) and act upon a regulated flow of the physical medium (2) forming the refrigerant physical medium (3) in a refrigerating chamber (1), at least one first closed circuit for the circulation of the refrigerant physical medium (3), at least one system for regulating its flow rate with the aid of a regulating device (7), at least one second closed circuit for the circulation of the heat-generating physical medium (6), at least one system for regulating its flow rate with the aid of a regulating device (8), at least one heat exchanger (1) bringing about the interaction between the refrigerant medium (3) and the heat-generating medium (6), and at least one vibration system (10) for decontaminating, disinfecting or sterilising the heat-generating physical medium.

5. A device according to Claim 4, **characterised in that** the said first and third vibration actions (13, 11) are regulated as regards their frequency and/or amplitude as a function of the flow rates of the refrigerant medium (3) and the heat-generating medium (6).
